# EUROPEAN PATENT SPECIFICATION

(11) **EP 4 244 604 B1**
(45) Date of publication and mention of the grant of the patent: **10.07.2024**
(21) Application number: 21730653.9
(22) Date of filing: 12.05.2021
(51) Int. Cl.: G01N 21/69, G01N 21/76, B01L 3/00, G01N 33/543

(54) **PORTABLE DEVICE FOR DETECTING AND QUANTIFYING A CONCENTRATION OF A MARKER PRESENT IN A SAMPLE OF BIOLOGICAL FLUID**
TRAGBARE VORRICHTUNG ZUM NACHWEIS UND ZUR QUANTIFIZIERUNG EINER KONZENTRATION EINES MARKERS IN EINER PROBE EINER BIOLOGISCHEN FLÜSSIGKEIT
DISPOSITIF PORTABLE DE DÉTECTION ET DE QUANTIFICATION D'UNE CONCENTRATION D'UN MARQUEUR PRÉSENT DANS UN ÉCHANTILLON DE FLUIDE BIOLOGIQUE

(30) Priority: 11.11.2020 IT 202000026924
(43) Date of publication of application: 20.09.2023
(73) Proprietor: RHAZES S.R.L., 34149 Trieste (IT)
(72) Inventor: KATOUZIANTEHRANI MOGHADAM, Fouroghalzaman, 34136 Trieste (IT)
(74) Representative: Bellomia, Paolo
(86) International application number: PCT/IB2021/054032
(87) International publication number: WO 2022/101686

(56) References cited:
- EP-A2- 2 284 536
- US-A1- 2004 189 311
- DELANEY JACQUI L. ET AL: "Electrogenerated Chemiluminescence Detection in Paper-Based Microfluidic Sensors", ANALYTICAL CHEMISTRY, vol. 83, no. 4, 15 February 2011 (2011-02-15), US, pages 1300 - 1306, XP055821539, ISSN: 0003-2700, DOI: 10.1021/ac102392t

## Description

The present invention relates to a portable device for detecting and quantifying a concentration of a marker present in a sample of biological fluid.

In particular, the present invention relates to a highly sensitive portable device for detecting and quantifying a concentration of a marker present in a biological fluid sample which is capable of detecting and determining concentrations of the marker up to 0.01 ng/L.

Such a device finds particular application in the medical and personal care fields, for quantifying the concentration of a marker, for example TROPONIN I, in a user's blood or plasma sample.

As is known, cardiovascular diseases, and especially acute coronary syndromes, are among the most common diseases and currently represent the cause of about half of all deaths in the Western world. Conditions related to cardiovascular diseases cost the European Union around 192 billion euros annually, representing a significant financial burden on healthcare resources.

One of the most dangerous forms of acute coronary syndromes is myocardial infarction, defined as necrosis of the cardiac myocytes following prolonged ischemia. Since myocardial infarction causes irreversible damage to the heart, a patient suspected of having such a disease must be diagnosed as quickly and efficiently as possible.

To this end, various tests are performed, such as the electrocardiogram and the measurement of specific biomarkers in the patient's blood.

However, for hospital admissions related to acute coronary syndromes, up to 50% of patients show an EKG reading which is considered normal or contains ambiguous information. Therefore, the assessment of the variation in the concentration of cardiac disease markers (referred to below as cardiac markers) is currently the most accurate source of information to allow the doctor to make an informed decision on the appropriate treatment for the patient.

The internationally recognized guidelines for the diagnosis of cardiac markers recommend a detection of TROPONIN I concentration in the blood starting from 5 ng/L in the blood sample and also recommend a response time for reporting results which is less than 60 minutes. In other words, the report of the tests carried out on the patient must be available within 60 minutes of the patient's admission.

However, such a guideline in terms of response time has proved problematic, as less than 25% of hospitals have successfully achieved the aforesaid goal.

It follows that, generally, the patient must wait longer than 60 minutes before being able to receive adequate healthcare treatment, putting his health at risk for the aforesaid reasons.

Furthermore, the patient's waiting inside the healthcare facility, in addition to being risky and unpleasant for the same patient, involves a further series of disadvantages, such as the possible filling of specific spaces in the healthcare facility or the financial burden deriving from the patient's prolonged stay.

Therefore, a strongly felt need in the sector is that of being able to rapidly measure the concentrations of markers, especially cardiac markers, so as to be able to efficiently initiate the healthcare treatment.

To meet this need, some point-of-care (POCT) devices have been made commercially available for identifying and determining the concentration of cardiac markers.

The expression "point-of-care devices" refers to all those devices allowing tests which can be performed near the patient and/or in the place where specific healthcare is provided and which simultaneously allow to promptly provide results.

In other words, such devices allow the patient to perform the tests in the doctor's office, in an ambulance, at home or in the hospital and allow healthcare personnel to obtain results quickly.

Consequently, Point-of-Care Testing is currently the most suitable organizational solution for correct and effective decentralized diagnostics, with constant control of the analytical quality by an analysis laboratory.

There are currently several Point-of-Care devices on the market intended for the diagnosis of myocardial infarction which operate by determining the concentration of cardiac markers, such as TROPONIN I.

Such devices for detecting and quantifying TROPONIN I are based on different analytical techniques.

For example, devices based on the electrochemical technique, on the detection technique by fluorescence spectroscopy or on the detection technique by infrared spectroscopy (FTIR) are known.

However, such devices have a series of disadvantages which make the use thereof still not very efficient.

First of all, none of the devices is capable of complying with the recommendations from the guidelines for the diagnosis of cardiac markers, as such devices have a TROPONIN I detection limit in a blood sample of not less than 20ng/L, i.e., almost five times greater than the established value.

A further disadvantage of such devices is related to the large volumes (> 100 µL) of biological sample required for the normal analysis operations and/or the need to pre-treat the biological sample (for example the separation of plasma or serum from the blood sample).

Another disadvantage is the cost of the devices, which make the purchase thereof prohibitive.

Such factors make the devices usable exclusively by healthcare professionals. US 2004/0189311 A1 and Delaney Jacqui L. ET AL "Electrogenerated Chemiluminescence Dectection in Paper-Based Microfluidic Sensors", Analytical Chemistry, vol. 83, no. 4, 15 February 2011, pages 1300-1306*,* disclose examples of known portable devices for detecting substances, wherein EP 2 284536 A2 discloses an electrochemiluminescence testing.

In this context, the technical task underlying the present invention is to propose a portable device for detecting and quantifying a concentration of a marker present in a biological fluid sample which overcomes the drawbacks of the prior art mentioned above.

In particular, it is an object of the present invention to provide a portable device for detecting and quantifying a concentration of a marker present in a biological fluid sample which can comply with the criteria recommended by the guidelines regarding detection sensitivity and precision.

It is a further object of the present invention to provide a portable device for detecting and quantifying a concentration of a marker present in a biological fluid sample which can be easily used even by unskilled personnel and/or by the patient himself/herself.

It is a further object of the present invention to provide a portable device for detecting and quantifying a concentration of a marker present in a biological fluid sample which is capable of providing results rapidly.

It is a further object of the present invention to provide a portable device for detecting and quantifying a concentration of a marker present in a biological fluid sample which is precise and reliable.

It is a further object of the present invention to provide a portable device for detecting and quantifying a concentration of a marker present in a biological fluid sample which requires a small volume of biological sample for correct operation.

It is a further object of the present invention to provide a portable device for detecting and quantifying a concentration of a marker present in a biological fluid sample which is easily and comfortably transportable, in particular by the user himself.

It is a further object of the present invention to provide a portable device for detecting and quantifying a concentration of a marker present in a biological fluid sample which is easily available on the market and which has a low cost.

The specified technical task and objects are substantially achieved by a portable device for detecting and quantifying a concentration of a marker present in a biological fluid sample which is easy to use, comprising the technical features set out in one or more of the appended claims.

In particular, the specified technical task and objects are substantially achieved by a portable device for detecting and quantifying a concentration of a marker, for example TROPONIN I, present in a biological fluid sample, comprising a cartridge adapted to receive the biological fluid sample and comprising at least one reactive agent configured to bind to the marker, defining an electrochemiluminescent solution. The cartridge is preferably coupled to an electrode cell configured to supply electrical energy to the electrochemiluminescent solution so as to trigger an electrochemiluminescence reaction of the electrochemiluminescent solution, generating a light signal representative of a concentration value of the marker in the biological fluid sample. The device further comprises an analyzer, connectable to the cartridge and configured to receive and analyze at least one property of the light signal so as to quantify a concentration of the marker in the biological fluid.

Further features of the present invention will become more apparent from the following indicative and thus non-limiting description of a preferred, but not exclusive, embodiment of such a device, as shown in the accompanying drawings, in which:
- figures 1A and 1B respectively show a diagrammatic perspective view and a diagrammatic top view of an embodiment of a cartridge for a portable device for detecting and quantifying a concentration of a marker present in a biological fluid sample in accordance with the present invention;
- figure 2 shows a diagrammatic section of the cartridge in figure 1; and
- figure 3 shows an embodiment diagram of an analyzer for the portable device for detecting and quantifying a concentration of a marker present in a biological fluid sample according to the present invention, during a use configuration in which the cartridge is connected to the analyzer.

With reference to the accompanying drawings, reference numeral 1 generally indicates a portable device for detecting and quantifying a concentration of a marker present in a biological fluid sample.

In the present description, the expression "biological fluid sample" is intended as human biomaterial, substantially in liquid form, sampled or which can be sampled from a subject.

Preferably, the expression "biological fluid sample" refers to a blood or plasma sample.

According to a preferred embodiment of the device 1, it finds application for detecting and quantifying a concentration of the marker TROPONIN I in blood or plasma.

However, according to alternative embodiments, which are not excluded from the scope of the present patent application, such a device 1 can be advantageously used for detecting and quantifying a concentration of a generic marker in a biological fluid sample, such as blood, plasma, saliva and urine.

For example, such a device can be used as a serological test for detecting and quantifying the antibodies produced in the event of infection due to the presence of a virus belonging to the coronavirus family.

Substantially, the device 1 comprises a cartridge 100 and an analyzer 200. The cartridge 100 is configured to receive the biological fluid sample and comprises at least one reactive agent configured to bind to the marker, defining an electrochemiluminescent solution.

In the present description, the term "bind" is intended as the formation of a bond, preferably of a chemical nature, which holds the at least one reactive agent and the marker together.

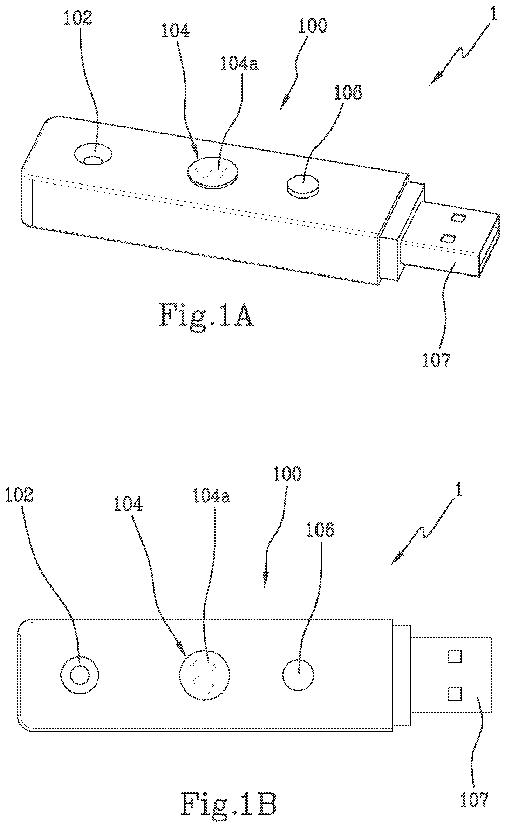

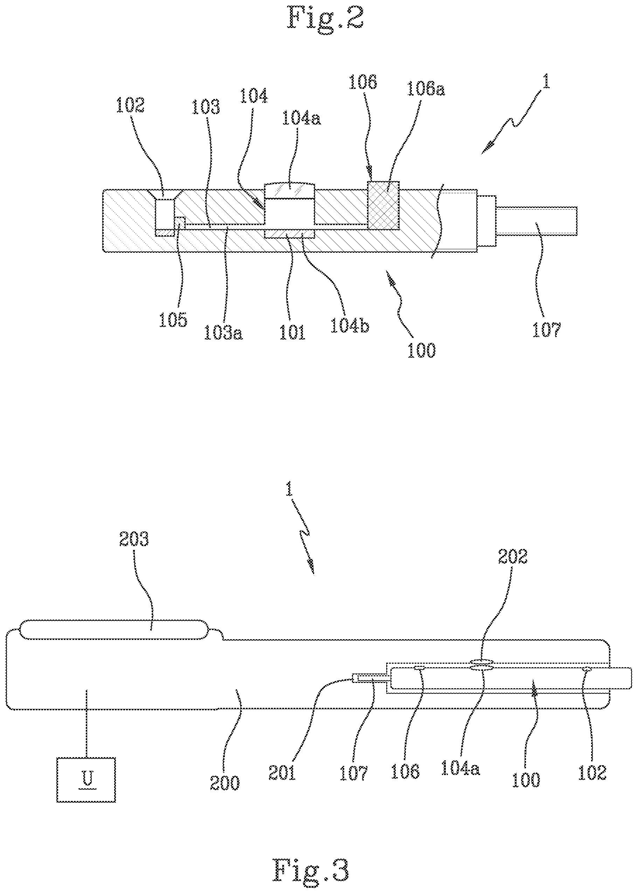

Figures 1A, 1B and 2 show an exemplary and non-limiting embodiment of the cartridge 100.

In such an embodiment, the cartridge 100 has a substantially parallelepiped shape.

The cartridge 100 is made of rigid material, preferably of polymeric material, even more preferably of polyester.

According to an aspect of the present invention, the cartridge 100 has a length between 4 cm and 8 cm, preferably 6 cm, a width between 1.5 cm and 2.5 cm, preferably 2 cm, and a thickness between 0.6 cm and 1.5 cm, preferably 0.8 cm.

Advantageously, the reduced size of the cartridge 100 allow the user to comfortably carry one or more cartridges 100 therewith.

Advantageously, moreover, such size allow the user to be able to store, for example in his own home, a plurality of cartridges 100 in a limited space, in order to have a supply always available in case of need.

The cartridge 100 can comprise a chamber 102 for receiving the biological fluid sample, preferably with a conical or frustoconical shape, adapted to collect the biological fluid sample.

Said receiving chamber 102 can further comprise a sampling element, not illustrated, configured to achieve an extraction of the biological fluid sample from a user when in contact with the user. For example, the sampling element can comprise a fingerstick or a lancing device.

Alternatively, the receiving chamber 102 can be configured to receive the biological fluid sample by means of a known alternative instrument, such as a transfer pipette or any equivalent instrument.

The cartridge 100 can comprise a first and a second reaction chamber, indicated in the figures with the respective reference numerals 103 and 104.

The first reaction chamber 103 is arranged in fluid communication with the receiving chamber 102 and comprises a first reactive agent configured to bind to the marker of the biological fluid sample.

Preferably, such a first reactive agent can be a secondary antibody configured to bind to TROPONIN I.

According to a first embodiment, the first reaction chamber 103 comprises a microfluidic channel 103a, interposed between the receiving chamber 102 and the second reaction chamber 104, and configured for a transport of the biological fluid sample from the receiving chamber 102 to the second reaction chamber 104.

According to such an embodiment, the first reaction chamber 103 is also at least partially coincident with the receiving chamber 102.

Preferably the first reactive agent is applied to a bottom wall of the receiving chamber 102 and is configured to detach from the bottom wall when in contact with the biological fluid sample.

In other words, the biological fluid sample is received in the receiving chamber 102 where it comes into contact with the first reactive agent, applied to the bottom of the receiving chamber 102, which binds to the marker detaching itself from the bottom walls and defining the aforesaid electrochemiluminescent solution.

A chemical and/or physical barrier 105 can also be present, interposed between the receiving chamber 102 and the second reaction chamber 104, configured to break apart after a certain period of time from a first contact between the barrier 105 and the biological fluid sample so as to slow down a movement of the biological fluid sample from the receiving chamber 102 to the second reaction chamber 104. Preferably, such a period of time is between 2 minutes and 10 minutes. Even more preferably, the period of time is equal to 5 minutes.

Such a barrier 105 is preferably operatively arranged before the microfluidic channel 103a.

Advantageously, by virtue of such a barrier 105, the first reactive agent has time to bind to the marker before the biological fluid sample reaches the second reaction chamber 104 through the microfluidic channel 103a. According to a further embodiment, the first reactive agent is preferably applied at least on the walls of the microfluidic channel 103a and is configured to detach from the walls when in contact with the biological fluid sample.

Furthermore, in such an embodiment, the barrier 105 is operatively arranged in a joining portion between the microfluidic channel 103a and the second reaction chamber 104. In other words, the barrier 105 is operatively arranged near an end of the microfluidic channel 103a connected and facing the second reaction chamber 104.

Such a microfluidic channel 103a can have a circular, square or rectangular section and has a width having a value between 40 µm and 500 µm, preferably equal to 80 µm.

Advantageously, the size of the microfluidic channel 103a causes a movement of the biological fluid sample which occurs substantially by capillarity.

It follows that the movement of the biological fluid sample inside the microfluidic channel 103a is slow enough to allow an optimal mixing of the first reactive agent with the biological fluid sample itself, defining the aforementioned electrochemiluminescent solution.

Functionally, in other words, the biological fluid sample from the receiving chamber 102 enters by capillarity inside the microfluidic channel 103a where it comes into contact with the first reactive agent which binds to the marker, detaching itself from the walls of the microfluidic channel and defining the aforesaid electrochemiluminescent solution.

According to a further embodiment, the first reactive agent can be applied both on the walls of the microfluidic channel 103a and on the bottom wall of the receiving chamber 102.

The second reaction chamber 104, arranged in fluid communication with the first reaction chamber 103, comprises a second reactive agent configured to also bind to the biological fluid sample marker.

Preferably, such a first reactive agent can be a primary antibody configured to bind to TROPONIN I.

Advantageously, the first and the second reactive agents are stable and non-toxic.

The cartridge 100 further comprises an electrode cell 101.

Such an electrode cell 101 is preferably inserted in the second reaction chamber 104.

Even more preferably, the electrode cell 101 is operatively arranged in a bottom portion 104b of the second reaction chamber 104.

In other words, the second reaction chamber 104 defines, together with the electrode cell 101, an electrochemical cell.

The electrode cell 101 is configured to supply electrical energy to the electrochemiluminescent solution so as to trigger an electrochemiluminescence reaction of the electrochemiluminescent solution capable of generating a light signal representative of a concentration of the marker in the biological fluid sample.

Preferably, the cartridge 100 is therefore disposable.

Structurally, the electrode cell 101 can comprise a working electrode, a reference electrode and an auxiliary electrode.

Preferably, the second reactive agent is applied to the working electrode.

At a functional level, the electrode cell 101 is preferably configured to generate a potential difference between distinct points, and in particular between the working electrode and the reference electrode, so as to cause a chemical reaction in the second reaction chamber 104. Advantageously, by using a three-electrode cell 101 to activate the electrochemiluminescence reaction, it is possible to precisely control the reaction.

In other words, by means of the energy supplied by the electrode cell 101, an electrochemiluminescent reaction occurs inside the second reaction chamber 104.

According to a further structural aspect, the working electrode can be made of vitreous carbon or gold or platinum, the reference electrode can be made of silver or silver chloride while the auxiliary electrode can be made of vitreous carbon or gold or platinum.

The cartridge 100 can further comprise a washing chamber 106, arranged in fluid communication with the second reaction chamber 104 and comprising an absorption element 106a configured to absorb and remove an excess portion of the electrochemiluminescent solution from the second reaction chamber 104.

By virtue of such a washing chamber 106 it is therefore possible to normalize the quantity of electrochemiluminescent solution necessary for the electrochemiluminescence reaction.

The excess portion can be a portion of the excess biological fluid sample and/or a portion of the first reactive agent not bound to the marker and/or a portion of the second reactive agent not bound to the marker. Preferably, the washing chamber 106 absorbs the electrochemiluminescent solution component not bound to the electrode cell.

Preferably, the washing chamber 106 is operatively arranged in a position opposite to the first receiving chamber 103 with respect to the second reaction chamber 104.

In other words, the second reaction chamber 104 is interposed between the washing chamber 106 and the first reaction chamber 103.

Preferably, the absorption element 106a is made of hydrophilic material. Even more preferably, the absorption element 106a is made of cellulose diacetate.

The electrode cell 101 is powered by an electric generator.

In this regard, the cartridge 101 can comprise a connector 107, preferably a normalized connector of the hot swap type, even more preferably a USB connector, configured for a reversible coupling with a source of electricity. In other words, such a connector 107 acts as a generator for the electrode cell 101.

Such a connector 107 can be applied to any compatible interface which is suitable for supplying electrical energy.

Preferably, the connector 107 can be applied directly to a receiving portion 201 of the analyzer 200, as will be seen in the following of the present description. In other words, in such an embodiment, the connector 107 is configured to activate the electrode cell 101 when the cartridge is connected to the analyzer 200.

As seen in the accompanying drawings, the second reaction chamber 104 comprises a covering element 104a, transparent to the electromagnetic radiation generated by the electrochemiluminescence reaction so as to allow the reception of the light signal by the analyzer 200.

In the present description, the expression "transparent to electromagnetic radiation" means that the covering element 104a allows the passage of the light signal produced by the electrochemiluminescence reaction.

Said covering element 104a is operatively arranged in a position opposite to the electrode cell 101 with respect to the second reaction chamber 104. In other words, the second reaction chamber 104 is at least partially delimited at the bottom by the electrode cell 101 operatively arranged in the bottom portion 104b and is delimited at the top by the covering element 104a.

Advantageously, such a covering element 104a has the dual function of protecting the second reaction chamber 104 and allowing the electromagnetic radiation of the light signal to reach the analyzer 200 which is, in use, operatively arranged in a position such as to be able to receive the light signal.

In particular, the analyzer 200 is connectable or connected to the cartridge 100 to receive and analyze at least one property of the light signal so as to quantify a concentration value of the marker in the biological fluid. Advantageously, the electrochemiluminescence technique allows to optimally analyze a large variety of biological samples, such as blood, plasma, serum, saliva, urine and cellular supernatant.

Furthermore, the sensitivity and precision of such a system are very high, allowing the detection and determination of marker concentrations up to 0.01 ng/L.

According to a preferred embodiment, illustrated by way of non-limiting example in figure 3, the analyzer 200 can comprise an optical sensor 202, arranged above the covering element 104a and oriented in the direction of the covering element 104a when the cartridge 100 is connected to the analyzer 200.

The optical sensor 202 is configured to measure a light intensity value of the light signal emitted by the electrochemiluminescence reaction.

The analyzer 200 can comprise a converter of the light signal captured by the optical sensor so as to convert the light signal of an analog nature into a digital one.

The analyzer 200 further comprises a control unit "U", connected to the optical sensor 202 and configured to determine the concentration of the marker as a function of the light intensity value measured by the optical sensor 202 and to generate information content representative of the concentration of said marker in said biological fluid sample.

Preferably, by analyzing the light signal coming from the optical sensor 202, the control unit "U" determines the quantification of the presence of the marker as a function of the light intensity of the light signal itself.

Functionally, the at least one reactive agent binds to the marker, generating an electrochemiluminescent solution which is activated by the electrode cell 101. The more marker present in the biological fluid sample, the more the electrochemiluminescent solution will emit photons. Consequently, by measuring the properties of the light signal emitted by the electrochemiluminescent solution, the analyzer 200 will be capable of determining the concentration of the marker since it is proportional to the electrochemiluminescent solution and therefore to the intensity of the light signal.

The analyzer 200 can further comprise a video and/or audio emission system 203 connected to the control unit "U" to receive the information content and configured to emit a visual and/or sound signal corresponding to the information content, so that the user can receive a visual/audible feedback of the measurement made.

For example, the video and/or audio emission system 203 can comprise at least one of a display, preferably digital, and at least one speaker.

According to an aspect of the present invention, the analyzer 200 can further comprise a communication system configured to transmit the information content to an external receiving unit.

For example, the analyzer 200 can comprise a wireless communication system for remote communication.

For example, if the device 1 is used on a patient during transport by ambulance, the test result can be sent directly to the healthcare facility where the patient is directed, so that he can immediately be subjected to a specific treatment.

Also, in the situation where the device 1 is used in one's home, the device sends the result of the test to the healthcare facility where the device user is being treated.

The analyzer 200 can have compact size so that it can be transported by a user.

According to a further aspect, the analyzer 200 can be powered by a power supply connectable to the electrical grid and/or have a support battery thereof and/or have a rechargeable battery.

According to a further aspect, the device 1 can comprise a plurality of cartridges 100 (a set or a kit) reversibly connectable to the analyzer 200, each cartridge 100 comprising a respective reactive agent configured to bind to a respective marker.

Advantageously, such a device 1 can thereby be used for detecting and determining the concentration of a plurality of markers.

Advantageously, according to an embodiment, the device 1 can be used for detecting a marker identifying the presence of a virus belonging to the coronavirus family.

Advantageously, according to an embodiment, the device 1 can be used for detecting the antibodies produced following infection due to the presence of a virus belonging to the coronavirus family.

For example, the device 1 can be advantageously used for detecting and quantifying a viral load of the severe acute respiratory syndrome from coronavirus 2 present in a biological fluid sample.

The present invention achieves the suggested objects, overcoming the drawbacks reported in the prior art.

Advantageously, the portable device 1 for detecting and quantifying a concentration of a marker present in a biological fluid sample can be easily used even by unskilled personnel and/or by the user himself.

Such a result is achieved by virtue of the structure of the cartridge 100 which determines the intuitive use thereof and by virtue of the analyzer 200 which operates in a completely automated manner and also allows rapid feedback to be provided to the user.

Advantageously, in fact, the portable device 1 for detecting and quantifying a concentration of a marker present in a biological fluid sample is capable of providing the results rapidly, precisely and by means of a low volume of biological fluid sample.

Such a result is achieved by virtue of the technical features of the cartridge 100 which allow triggering the chemiluminescence reaction in a few minutes and with a small biological fluid sample and by virtue of the technical features of the analyzer 200 which allow a very rapid and precise analysis by exploiting the electrochemiluminescence reaction.

A further object achieved is that of providing a portable device 1 for detecting and quantifying a concentration of a marker present in a biological fluid sample which is easily and comfortably transportable, in particular by the user himself. Such a result is achieved by virtue of the small size of the cartridge 100 and the analyzer 200.

## Claims

1. A portable device (1) for detecting and quantifying a concentration of a marker, for example TROPONIN I, present in a sample of biological fluid, said device comprising:
- a cartridge (100) adapted to receive said biological fluid sample and comprising at least one reactive agent configured to bind to the marker defining an electrochemiluminescent solution; said cartridge (100) comprising an electrode cell (101) configured to supply electrical energy to the electrochemiluminescent solution so as to trigger an electrochemiluminescence reaction of said electrochemiluminescent solution, generating a light signal representative of a concentration value of said marker in said biological fluid sample;
- an analyzer (200), connected or connectable to said cartridge (100) and configured to receive said light signal and analyze at least one property of said light signal so as to quantify a concentration of said marker in the biological fluid;
wherein said cartridge (100) comprises:
- a chamber (102) for receiving said biological fluid sample, preferably with a conical or frustoconical shape, adapted to collect said biological fluid sample;
- a first reaction chamber (103), arranged in fluid communication with said receiving chamber (102) and comprising a first reactive agent configured to bind to said marker of said biological fluid sample;
- a second reaction chamber (104), arranged in fluid communication with said first reaction chamber (103), said reaction chamber (103) being interposed between the receiving chamber (102) and the second reaction chamber (104), and comprising a second reactive agent configured to bind to said marker of said biological fluid sample; said second reaction chamber (104) comprising a covering element (104a) transparent to the electromagnetic radiation generated by said electrochemiluminescence reaction to allow the reception of the electromagnetic radiation by the analyzer (200);
**characterized in that** said cartridge (100) comprises a washing chamber (106), arranged in fluid communication with said second reaction chamber (104) and comprising an absorption element (106a) configured to absorb and remove from said second reaction chamber (104) an excess portion of said electrochemiluminescent solution; said excess portion of said electrochemiluminescent solution being a portion of said excess biological fluid sample and/or a portion of said first reactive agent not bound to said marker and/or a portion of said second reactive agent not bound to said marker; preferably said absorption element (106a) being made of hydrophilic material, even more preferably said absorption element (106a) being made of cellulose diacetate.

2. A device (1) according to claim 1, wherein said electrode cell (101) is operatively arranged in a bottom portion (104b) of said second reaction chamber (104), said electrode cell (101) preferably being a cell of the three-electrode type and being configured to generate a potential difference between two of said three electrodes.

3. A device (1) according to claim 1 or 2, wherein said first reaction chamber (103) comprises a microfluidic channel (103a), interposed between said receiving chamber (102) and said second reaction chamber (104), configured for a transport of said biological fluid sample from said receiving chamber (102) to said second reaction chamber (104); preferably said first reactive agent being applied at least on the walls of said microfluidic channel (103a) and being configured to detach from said walls when in contact with said biological fluid sample.

4. A device (1) according to any one of the preceding claims 1 to 3, wherein said first reaction chamber (103) is at least partially coincident with said receiving chamber (102); preferably said first reactive agent being applied to a bottom wall of said receiving chamber (102) and being configured to detach from said bottom wall when in contact with said biological fluid sample.

5. A device (1) according to any one of the preceding claims 1 to 4, wherein said cartridge (100) comprises a chemical and/or physical barrier (105), interposed between the receiving chamber (102) and the second reaction chamber (104), configured to break apart after a certain period of time from a first contact between said barrier (105) and said biological fluid sample so as to slow down a movement of said biological fluid sample from said receiving chamber (102) to said second reaction chamber (104); preferably said period of time being between 2 minutes and 10 minutes, even more preferably said period of time being equal to 5 minutes.

6. A device (1) according to any one of the preceding claims 1 to 5, wherein said cartridge (100) comprises a connector (107), preferably a standard connector of the hot swap type, even more preferably a USB connector, configured for a reversible coupling with a respective receiving portion (201) of said analyzer (200); said connector (107) being configured to activate said electrode cell (101) when said cartridge (100) is connected to said analyzer (200).

7. A device (1) according to any one of the preceding claims 1 to 6, wherein said analyzer (200) comprises:
- an optical sensor (202), arranged above said covering element (104a) and oriented in the direction of said covering element (104a) when said cartridge (100) is connected to said analyzer (200), said optical instrument (202) being configured to measure a light intensity value of the light signal emitted by said electrochemiluminescence reaction;
- a control unit (U), connected to said optical sensor (202) and configured to determine the concentration of said marker as a function of the light intensity value measured by the optical sensor (202); said control unit (U) being further configured to generate information content representative of the concentration of said marker in said biological fluid sample; and
- a video and/or audio emission system (203) connected to said control unit (U) to receive said information content and configured to emit a visual and/or acoustic signal corresponding to said information content.

8. A device (1) according to claim 7, wherein said analyzer (200) comprises a communication system configured to transmit said information content to an external receiving unit.

9. A device (1) according to any one of the preceding claims, wherein said electrode cell (101) comprises a working electrode, a reference electrode and an auxiliary electrode; said working electrode preferably being made of vitreous carbon or gold or platinum; said reference electrode preferably being made of silver or silver chloride; said auxiliary electrode preferably being made of vitreous carbon or gold or platinum.

10. A device (1) according to any one of the claims, wherein said receiving chamber (102) comprises a sampling element configured to perform an extraction of said biological fluid sample from a user when in contact with the user, preferably said sampling element comprising a fingerstick or a lancing device.

11. A device according to any one of the preceding claims, comprising a plurality of cartridges (100) reversibly connectable to the analyzer (200), each cartridge comprising a respective reactive agent configured to bind to a respective marker.

12. Use of a device according to one or more of the preceding claims for the detection of a marker identifying the presence of a virus belonging to the coronavirus family.

## Patentansprüche

1. Tragbare Vorrichtung (1) zum Nachweis und zur Quantifizierung einer Konzentration eines Markers, beispielsweise TROPONIN I, in einer Probe einer biologischen Flüssigkeit, wobei die Vorrichtung umfasst:
- eine Patrone (100), die zum Empfang der biologischen Flüssigkeitsprobe angepasst ist und mindestens ein reaktives Mittel umfasst, das ausgelegt ist, um an den Marker zu binden, der eine elektrochemilumineszierende Lösung definiert; wobei die Patrone (100) eine Elektrodenzelle (101) umfasst, die ausgelegt ist, um der elektrochemilumineszierenden Lösung elektrische Energie zuzuführen, um eine Elektrochemilumineszenzreaktion der elektrochemilumineszierenden Lösung auszulösen, wodurch ein Lichtsignal erzeugt wird, das für einen Konzentrationswert des Markers in der biologischen Flüssigkeitsprobe repräsentativ ist;
- einen Analysator (200), der mit der Patrone (100) verbunden oder verbindbar ist und ausgelegt ist, um das Lichtsignal zu empfangen und mindestens eine Eigenschaft des Lichtsignals zu analysieren, um eine Konzentration des Markers in der biologischen Flüssigkeit zu quantifizieren;
wobei die Patrone (100) Folgendes umfasst:
- eine Kammer (102) zum Empfangen der biologischen Flüssigkeitsprobe, vorzugsweise mit einer konischen oder kegelstumpfförmigen Form, die angepasst ist, um die biologische Flüssigkeitsprobe zu sammeln;
- eine erste Reaktionskammer (103), die in Fluidkommunikation mit der Empfangskammer (102) angeordnet ist und ein erstes reaktives Mittel umfasst, das ausgelegt ist, um an den Marker der biologischen Flüssigkeitsprobe zu binden;
- eine zweite Reaktionskammer (104), die in Fluidkommunikation mit der ersten Reaktionskammer (103) angeordnet ist, wobei die Reaktionskammer (103) zwischen der Empfangskammer (102) und der zweiten Reaktionskammer (104) angeordnet ist und ein zweites reaktives Mittel umfasst, das ausgelegt ist, um an den Marker der biologischen Flüssigkeitsprobe zu binden; wobei die zweite Reaktionskammer (104) ein Abdeckelement (104a) umfasst, das für die durch die Elektrochemilumineszenzreaktion erzeugte elektromagnetische Strahlung transparent ist, um den Empfang der elektromagnetischen Strahlung durch den Analysator (200) zu ermöglichen;
**dadurch gekennzeichnet, dass** die Patrone (100) eine Waschkammer (106) umfasst, die in Fluidkommunikation mit der zweiten Reaktionskammer (104) angeordnet ist und ein Absorptionselement (106a) umfasst, das ausgelegt ist, um einen überschüssigen Teil der elektrochemilumineszierenden Lösung zu absorbieren und aus der zweiten Reaktionskammer (104) zu entfernen; wobei der überschüssige Teil der elektrochemilumineszierenden Lösung ein Teil der überschüssigen biologischen Flüssigkeitsprobe und/oder ein Teil des ersten reaktiven Mittels, der nicht an den Marker gebunden ist, und/oder ein Teil des zweiten reaktiven Mittels, der nicht an den Marker gebunden ist, ist; wobei das Absorptionselement (106a) vorzugsweise aus hydrophilem Material gefertigt ist, wobei das Absorptionselement (106a) noch bevorzugter aus Cellulosediacetat gefertigt ist.

2. Vorrichtung (1) nach Anspruch 1, wobei die Elektrodenzelle (101) betriebswirksam in einem Bodenabschnitt (104b) der zweiten Reaktionskammer (104) angeordnet ist, wobei die Elektrodenzelle (101) vorzugsweise eine Zelle des Drei-Elektroden-Typs ist und ausgelegt ist, um eine Potentialdifferenz zwischen zwei der drei Elektroden zu erzeugen.

3. Vorrichtung (1) nach Anspruch 1 oder 2, wobei die erste Reaktionskammer (103) einen mikrofluidischen Kanal (103a) umfasst, der zwischen der Empfangskammer (102) und der zweiten Reaktionskammer (104) angeordnet ist und für einen Transport der biologischen Flüssigkeitsprobe von der Empfangskammer (102) zu der zweiten Reaktionskammer (104) ausgelegt ist;
vorzugsweise wird das erste reaktive Mittel mindestens auf die Wände des mikrofluidischen Kanals (103a) aufgebracht und ist dazu ausgelegt, sich von den Wänden zu lösen, wenn es mit der biologischen Flüssigkeitsprobe in Kontakt steht.

4. Vorrichtung (1) nach einem der vorhergehenden Ansprüche 1 bis 3, wobei die erste Reaktionskammer (103) zumindest teilweise mit der Empfangskammer (102) zusammenfällt; wobei vorzugsweise das erste reaktive Mittel auf eine Bodenwand der Empfangskammer (102) aufgebracht wird und ist dazu ausgelegt, sich von der Bodenwand zu lösen, wenn es mit der biologischen Flüssigkeitsprobe in Kontakt steht.

5. Vorrichtung (1) nach einem der vorhergehenden Ansprüche 1 bis 4, wobei die Patrone (100) eine chemische und/oder physikalische Barriere (105) umfasst, die zwischen der Empfangskammer (102) und der zweiten Reaktionskammer (104) angeordnet ist und so ausgelegt ist, dass sie nach einer bestimmten Zeitspanne von einem ersten Kontakt zwischen der Barriere (105) und der biologischen Flüssigkeitsprobe auseinanderbricht, um eine Bewegung der biologischen Flüssigkeitsprobe von der Empfangskammer (102) zu der zweiten Reaktionskammer (104) zu verlangsamen; wobei die Zeitspanne vorzugsweise zwischen 2 Minuten und 10 Minuten liegt, noch bevorzugter die Zeitspanne gleich 5 Minuten ist.

6. Vorrichtung (1) nach einem der vorhergehenden Ansprüche 1 bis 5, wobei die Patrone (100) einen Verbinder (107), vorzugsweise einen Standardverbinder des Hot-Swap-Typs, noch bevorzugter einen USB-Verbinder, umfasst, der für eine reversible Kopplung mit einem jeweiligen Empfangsabschnitt (201) des Analysators (200) ausgelegt ist; wobei der Verbinder (107) ausgelegt ist, um die Elektrodenzelle (101) zu aktivieren, wenn die Patrone (100) mit dem Analysator (200) verbunden ist.

7. Vorrichtung (1) nach einem der vorhergehenden Ansprüche 1 bis 6, wobei der Analysator (200) umfasst:
- einen optischen Sensor (202), der über dem Abdeckelement (104a) angeordnet und in Richtung des Abdeckelements (104a) orientiert ist, wenn die Patrone (100) mit dem Analysator (200) verbunden ist, wobei das optische Instrument (202) dazu ausgelegt ist, einen Lichtintensitätswert des durch die Elektrochemilumineszenzreaktion emittierten Lichtsignals zu messen;
- eine Steuereinheit (U), die mit dem optischen Sensor (202) verbunden und ausgelegt ist, um die Konzentration des Markers als Funktion des von dem optischen Sensor (202) gemessenen Lichtintensitätswerts zu bestimmen; wobei die Steuereinheit (U) ferner ausgelegt ist, um Informationsinhalt zu erzeugen, der für die Konzentration des Markers in der biologischen Flüssigkeitsprobe repräsentativ ist; und
- ein Video- und/oder Audioemissionssystem (203), das mit der Steuereinheit (U) verbunden ist, um den Informationsinhalt zu empfangen, und ausgelegt ist, um ein visuelles und/oder akustisches Signal zu emittieren, das dem Informationsinhalt entspricht.

8. Vorrichtung (1) nach Anspruch 7, wobei der Analysator (200) ein Kommunikationssystem umfasst, das dazu ausgelegt ist, den Informationsinhalt an eine externe Empfangseinheit zu übertragen.

9. Vorrichtung (1) nach einem der vorhergehenden Ansprüche, wobei die Elektrodenzelle (101) eine Arbeitselektrode, eine Referenzelektrode und eine Hilfselektrode umfasst; wobei die Arbeitselektrode vorzugsweise aus glasartigem Kohlenstoff oder Gold oder Platin gefertigt ist; wobei die Referenzelektrode vorzugsweise aus Silber oder Silberchlorid gefertigt ist; wobei die Hilfselektrode vorzugsweise aus glasartigem Kohlenstoff oder Gold oder Platin gefertigt ist.

10. Vorrichtung (1) nach einem der Ansprüche, wobei die Empfangskammer (102) ein Probennahmeelement umfasst, das ausgelegt ist, um eine Extraktion der biologischen Flüssigkeitsprobe von einem Benutzer durchzuführen, wenn sie in Kontakt mit dem Benutzer ist, wobei das Probennahmeelement vorzugsweise einen Fingerstick oder eine Lanzette umfasst.

11. Vorrichtung nach einem der vorhergehenden Ansprüche, umfassend eine Vielzahl von Patronen (100), die reversibel mit dem Analysator (200) verbindbar sind, wobei eine jede Patrone ein jeweiliges reaktives Mittel umfasst, das ausgelegt ist, um an einen jeweiligen Marker zu binden.

12. Verwendung einer Vorrichtung nach einem oder mehreren der vorhergehenden Ansprüche zum Nachweis eines Markers, der das Vorhandensein eines zur Coronavirus-Familie gehörenden Virus identifiziert.

## Revendications

1. Dispositif portable (1) de détection et de quantification d'une concentration d'un marqueur, par exemple la TROPONINE I, présent dans un échantillon de fluide biologique, ledit dispositif comprenant :
- une cartouche (100) adaptée pour recevoir ledit échantillon de fluide biologique et comprenant au moins un agent réactif configuré pour se lier au marqueur définissant une solution d'électrochimiluminescence ; ladite cartouche (100) comprenant une cellule d'électrode (101) configurée pour fournir de l'énergie électrique à la solution d'électrochimiluminescence de façon à déclencher une réaction d'électrochimiluminescence de ladite solution d'électrochimiluminescence, générant un signal lumineux représentatif d'une valeur de concentration dudit marqueur dans ledit échantillon de fluide biologique ;
- un analyseur (200), relié ou pouvant être relié à ladite cartouche (100) et configuré pour recevoir ledit signal lumineux et analyser au moins une propriété dudit signal lumineux afin de quantifier une concentration dudit marqueur dans le fluide biologique ;
dans lequel ladite cartouche (100) comprend :
- une chambre (102) destinée à recevoir ledit échantillon de fluide biologique, de préférence de forme conique ou tronconique, adaptée pour recueillir ledit échantillon de fluide biologique ;
- une première chambre de réaction (103), disposée en communication fluidique avec ladite chambre de réception (102) et comprenant un premier agent réactif configuré pour se lier audit marqueur dudit échantillon de fluide biologique ;
- une deuxième chambre de réaction (104), disposée en communication fluidique avec ladite première chambre de réaction (103), ladite chambre de réaction (103) étant interposée entre la chambre de réception (102) et la deuxième chambre de réaction (104), et comprenant un deuxième agent réactif configuré pour se lier audit marqueur dudit échantillon de fluide biologique ; ladite deuxième chambre de réaction (104) comprenant un élément de recouvrement (104a) transparent au rayonnement électromagnétique généré par ladite réaction d'électrochimiluminescence pour permettre la réception du rayonnement électromagnétique par l'analyseur (200) ;
**caractérisé en ce que** ladite cartouche (100) comprend une chambre de lavage (106), disposée en communication fluidique avec ladite deuxième chambre de réaction (104) et comprenant un élément d'absorption (106a) configuré pour absorber et retirer de ladite deuxième chambre de réaction (104) une partie excédentaire de ladite solution d'électrochimiluminescence ; ladite partie excédentaire de ladite solution d'électrochimiluminescence est une partie dudit échantillon de fluide biologique excédentaire et/ou une partie dudit premier agent réactif non lié audit marqueur et/ou une partie dudit deuxième agent réactif non lié audit marqueur ; de préférence, ledit élément d'absorption (106a) étant constitué d'un matériau hydrophile, encore plus préférablement, ledit élément d'absorption (106a) est constitué de diacétate de cellulose.

2. Dispositif (1) selon la revendication 1, dans lequel ladite cellule d'électrode (101) est disposée de manière fonctionnelle dans une partie de fond (104b) de ladite deuxième chambre de réaction (104), ladite cellule d'électrode (101) étant de préférence une cellule du type à trois électrodes et étant configurée pour générer une différence de potentiel entre deux desdites trois électrodes.

3. Dispositif (1) selon la revendication 1 ou 2, dans lequel ladite première chambre de réaction (103) comprend un canal microfluidique (103a), interposé entre ladite chambre de réception (102) et ladite deuxième chambre de réaction (104), configuré pour un transport dudit échantillon de fluide biologique de ladite chambre de réception (102) à ladite deuxième chambre de réaction (104) ;
de préférence, ledit premier agent réactif étant appliqué au moins sur les parois dudit canal microfluidique (103a) et étant configuré pour se détacher desdites parois lorsqu'il est en contact avec ledit échantillon de fluide biologique.

4. Dispositif (1) selon l'une quelconque des revendications précédentes 1 à 3, dans lequel ladite première chambre de réaction (103) coïncide au moins partiellement avec ladite chambre de réception (102) ; de préférence, ledit premier agent réactif étant appliqué sur une paroi de fond de ladite chambre de réception (102) et est configuré pour se détacher de ladite paroi de fond lorsqu'il est en contact avec ledit échantillon de fluide biologique.

5. Dispositif (1) selon l'une quelconque des revendications précédentes 1 à 4, dans lequel ladite cartouche (100) comprend une barrière chimique et/ou physique (105), interposée entre la chambre de réception (102) et la deuxième chambre de réaction (104), configurée pour se rompre après un certain laps de temps à partir d'un premier contact entre ladite barrière (105) et ledit échantillon de fluide biologique de manière à ralentir un mouvement dudit échantillon de fluide biologique de ladite chambre de réception (102) vers ladite deuxième chambre de réaction (104) ; de préférence, ledit laps de temps est compris entre 2 et 10 minutes, encore plus préférablement, ledit laps de temps étant égal à 5 minutes.

6. Dispositif (1) selon l'une quelconque des revendications précédentes 1 à 5, dans lequel ladite cartouche (100) comprend un connecteur (107), de préférence un connecteur standard de type à permutation à chaud, encore plus préférablement un connecteur USB, configuré pour un couplage réversible avec une partie réceptrice (201) respective dudit analyseur (200) ; ledit connecteur (107) étant configuré pour activer ladite cellule d'électrode (101) lorsque ladite cartouche (100) est reliée audit analyseur (200).

7. Dispositif (1) selon l'une quelconque des revendications précédentes 1 à 6, dans lequel ledit analyseur (200) comprend :
- un capteur optique (202), disposé au-dessus dudit élément de recouvrement (104a) et orienté dans la direction dudit élément de recouvrement (104a) lorsque ladite cartouche (100) est reliée audit analyseur (200), ledit instrument optique (202) étant configuré pour mesurer une valeur d'intensité lumineuse du signal lumineux émis par ladite réaction d'électrochimiluminescence ;
- une unité de contrôle (U), reliée audit capteur optique (202) et configurée pour déterminer la concentration dudit marqueur en fonction de la valeur d'intensité lumineuse mesurée par le capteur optique (202) ; ladite unité de contrôle (U) étant de plus configurée pour générer un contenu d'informations représentatif de la concentration dudit marqueur dans ledit échantillon de fluide biologique ; et
- un système d'émission vidéo et/ou audio (203) relié à ladite unité de contrôle (U) pour recevoir ledit contenu d'informations et configuré pour émettre un signal visuel et/ou acoustique correspondant audit contenu d'informations.

8. Dispositif (1) selon la revendication 7, dans lequel ledit analyseur (200) comprend un système de communication configuré pour transmettre ledit contenu d'informations à une unité de réception externe.

9. Dispositif (1) selon l'une quelconque des revendications précédentes, dans lequel ladite cellule d'électrode (101) comprend une électrode de travail, une électrode de référence et une électrode auxiliaire ; ladite électrode de travail étant de préférence en carbone vitreux ou en or ou en platine ; ladite électrode de référence étant de préférence en argent ou en chlorure d'argent ; ladite électrode auxiliaire étant de préférence en carbone vitreux ou en or ou en platine.

10. Dispositif (1) selon l'une quelconque des revendications, dans lequel ladite chambre de réception (102) comprend un élément d'échantillonnage configuré pour effectuer une extraction dudit échantillon de fluide biologique d'un utilisateur lorsqu'il est en contact avec l'utilisateur, de préférence ledit élément d'échantillonnage comprenant un autopiqueur ou un dispositif à lancette.

11. Dispositif selon l'une quelconque des revendications précédentes, comprenant une pluralité de cartouches (100) pouvant être reliées de manière réversible à l'analyseur (200), chaque cartouche comprenant un agent réactif respectif configuré pour se lier à un marqueur respectif.

12. Utilisation d'un dispositif selon l'une ou plusieurs des revendications précédentes pour la détection d'un marqueur identifiant la présence d'un virus appartenant à la famille des coronavirus.
